# EUROPÄISCHE PATENTSCHRIFT

(11) **EP 1 663 947 B1**
(45) Veröffentlichungstag und Bekanntmachung des Hinweises auf die Patenterteilung: **16.01.2008**
(21) Anmeldenummer: 04764998.3
(22) Anmeldetag: 09.09.2004
(51) Int. Cl.: C07C 209/48, C07C 211/27

(54) **VERFAHREN ZUR HERSTELLUNG VON XYLYLENDIAMIN DURCH KONTINUIERLICHE HYDRIERUNG VON FLÜSSIGEM PHTHALODINITRIL**
METHOD FOR THE PRODUCTION OF DIAMINOXYLENE BY CONTINUOUS HYDROGENATION OF LIQUID PHTHALONITRILE
PROCEDE DE PRODUCTION DE XYLYLENEDIAMINE PAR HYDROGENATION CONTINUE DE PHTALODINITRILE LIQUIDE

(30) Priorität: 10.09.2003 DE 10341612; 10.09.2003 DE 10341633; 10.09.2003 DE 10341632; 10.09.2003 DE 10341615; 10.09.2003 DE 10341613; 10.09.2003 DE 10341614; 02.09.2004 DE 102004042947
(43) Veröffentlichungstag der Anmeldung: 07.06.2006
(73) Patentinhaber: BASF Aktiengesellschaft, 67056 Ludwigshafen (DE)
(72) Erfinder: HUGO, Randolf, 67246 Dirmstein (DE); WENZ, Kirsten, 68161 Mannheim (DE); WAMBSGANSS, Rolf, 76829 Landau (DE); JOURDAN, Sabine, 68163 Mannheim (DE); PREISS, Thomas, 67256 Weisenheim (DE)
(86) Internationale Anmeldenummer: PCT/EP2004/010063
(87) Internationale Veröffentlichungsnummer: WO 2005/026099

(56) Entgegenhaltungen:
- EP-A1- 0 449 089
- EP-A1- 1 279 661
- EP-A2- 1 193 244
- DE-B- 1 119 285
- DE-C- 902 616
- US-A- 3 574 754

## Beschreibung

Die vorliegende Erfindung betrifft ein Verfahren zur Herstellung von Xylylendiamin durch kontinuierliche Hydrierung von flüssigem Phthalodinitril an einem Heterogenkatalysator in Gegenwart von flüssigem Ammoniak in einem Reaktor.

Xylylendiamin (Bis(aminomethyl)benzol) ist ein nützlicher Ausgangsstoff, z.B. für die Synthese von Polyamiden, Epoxyhärtern oder als Zwischenstufe zur Herstellung von Isocyanaten.

Die Bezeichnung "Xylylendiamin" (XDA) umfasst die drei Isomere ortho-Xylylendiamin, meta-Xylylendiamin (MXDA) und para-Xylylendiamin.

Der Begriff "Phthalodinitril" (PDN) umfasst die drei Isomere 1,2-Dicyanbenzol = o-Phthalodinitril, 1,3-Dicyanbenzol = Isophthalodinitril = IPDN und 1,4-Dicyanbenzol = Terephthalodinitril.

Die Phthalodinitrile sind Feststoffe (z.B. schmilzt Isophthalodinitril (IPDN) bei 161°C) und weisen relativ schlechte Löslichkeiten in organischen Lösungsmitteln auf.

Die zweistufige Synthese von Xylylendiamin durch Ammonoxidation von Xylol und anschließender Hydrierung des erhaltenen Phthalodinitrils ist bekannt.
Unumgesetzte Dinitrile lassen sich nur sehr schwer vom XDA destillativ trennen.

US-A-4,482,741 (UOP Inc.) beschreibt die Hydrierung von PDN in Gegenwart von Ammoniak, einem spezifischen Katalysator und XDA als Lösungsmittel.

In MXDA beträgt die Löslichkeit von IPDN bei 70 °C ca. 20 Gew.-%.

EP-A2-1 193 247 und EP-A1-1 279 661 (beide Mitsubishi Gas Chem. Comp.) betreffen ein Verfahren zur Reinigung von Isophthalodinitril (IPDN) bzw. ein Verfahren zur Herstellung von reinem XDA.

EP-A2-1 193 244 (Mitsubishi Gas Chem. Comp.) beschreibt ein Verfahren zur Herstellung von XDA durch Hydrierung von Phthalodinitril, welches in einer vorherigen Stufe durch Ammonoxidation von Xylol synthetisiert wird, wobei das dampfförmige Produkt der Ammonoxidationsstufe direkt mit einem flüssigen organischen Lösungsmittel in Kontakt gebracht wird (Quench) und die erhaltene Quenchlösung oder -suspension der Hydrierung zugeführt wird.

Bevorzugte organische Lösungsmittel sind C₆-C₁₂ aromatische Kohlenwasserstoffe, wie Xylol und Pseudocumol (Spalte 6, Absatz [0027] und [0028]).

US-A-3,069,469 (California Research Corp.) lehrt als Lösungsmittel zur Hydrierung von aromatischen Nitrilen, wie PDN, aromatische Kohlenwasserstoffe, Xylol, Dioxan und aliphatische Alkohole.

DE-A-21 64 169 (Mitsubishi Gas Chemical Co., Inc.) beschreibt auf Seite 6, letzter Absatz, die Hydrierung von IPDN zu meta-XDA in Gegenwart eines Ni- und/oder Co-Katalysators in Ammoniak als Lösungsmittel.

GB-A-852,972 (Äquivalent: DE-A-11 19 285) (BASF AG) offenbart die Verwendung von Ammoniak und XDA als Lösungsmittel in der Hydrierung von PDN. Die Herstellung der Edukt-Lösung ausgehend von festem PDN erfolgt in einem Extraschritt in einem separaten Gefäß (vergl. Seite 2, Zeilen 119-120).

JP-A-2003-327563 (Mitsubishi Gas Chem. Co., Inc.) betrifft ein Verfahren zur Festbett-Hydrierung von aromatischen Dinitrilen, die als 1-10 Gew.-%ige Lösungen eingesetzt werden.

Die sechs deutschen Patentanmeldungen mit den Aktenzeichen 10341615.3, 10341632.3, 10341614.5, 10341633.1, 10341612.9 und 10341613.7 (BASF AG) vom 10.09.03 betreffen jeweils Verfahren zur Herstellung von XDA.

Die deutsche Patentanmeldung Nr. 102004042954.5 vom 02.09.04 (BASF AG) betrifft ein Verfahren zur Herstellung von XDA durch kontinuierliche Hydrierung von flüssigem Phthalodinitril an einem Heterogenkatalysator in Gegenwart von flüssigem Ammoniak in einem Reaktor, bei dem ein Teil des Reaktoraustrags als flüssiger Umlaufstrom kontinuierlich zum Reaktoreingang zurückgeführt wird (Kreislauffahrweise), in dem mittels einer Mischeinrichtung ein Strom einer Phthalodinitrilschmelze flüssig in den Umlaufstrom um den Hydrierreaktor zugefahren wird, wobei der Phthalodinitril-Umsatz im Reaktor bei einfachem Durchgang größer 99 % beträgt, und der Umlaufstrom zu größer 93 Gew.-% aus flüssigem Ammoniak und Xylylendiamin besteht und kein weiteres Lösungsmittel für Phthalodinitril enthält.

Bei den unterschiedlichen Verfahren zur Herstellung von Phthalodinitril fällt dieses als Feststoff oder gelöst in einem Lösungsmittel, z.B. Pseudocumol, oder als Schmelze an. Die Handhabung von Feststoffen ist üblicherweise schwierig und umständlich. Die Weiterverarbeitung in einem Lösungsmittel erfordert wegen der geringen Löslichkeit von Phthalodinitril in Lösungsmitteln wie o-Xylol, m-Xylol, p-Xylol, Pseudocumol, Mesitylen, Ethylbenzol oder Methylpyridin sehr große Lösungsmittelmengen, die nach der Hydrierung in der Regel destillativ abgetrennt werden müssen, was - entsprechend den großen Mengenströmen - große Apparate und einen hohen Energieaufwand erfordert. Alternativ ist eine Extraktion des PDNs mit Wasser mit anschlie ßender Destillation möglich. Auch hier ist der Energieaufwand groß, da das Wasser abdestilliert und das Lösungsmittel - zumindest im Teilstrom - regeneriert werden muss.

Der vorliegenden Erfindung lag die Aufgabe zugrunde, ein verbessertes wirtschaftliches Verfahren zur Herstellung von Xylylendiamin, insbesondere meta-Xylylendiamin, mit hoher Selektivität, Ausbeute und Raum-Zeit-Ausbeute (RZA) aufzufinden, welches bei mit Verfahren des Stands der Technik vergleichbaren Durchsätzen aufgrund verringerter Stoffströme, insbesondere Lösungsmittelströme, inkl. Rückführströme, verkleinerte und/oder weniger Apparate und Maschinen ermöglicht.

Demgemäß wurde ein Verfahren zur Herstellung von Xylylendiamin durch kontinuierliche Hydrierung von flüssigem Phthalodinitril an einem Heterogenkatalysator in Gegenwart von flüssigem Ammoniak in einem Reaktor gefunden, welches dadurch gekennzeichnet ist, dass mittels einer Mischeinrichtung ein Strom einer Phthalodinitrilschmelze flüssig mit einem Strom von flüssigem Ammoniak gemischt wird und die flüssige Mischung in den Hydrierreaktor gefahren wird.

Bevorzugt findet das erfindungsgemäße Verfahren Anwendung zur Herstellung von meta-Xylylendiamin (MXDA) durch Hydrierung von Isophthalodinitril (IPDN), welches insbesondere in einer vorherigen Stufe durch Ammonoxidation von meta-Xylol synthetisiert wurde.

Das geschmolzene Phthalodinitril kann beispielsweise aus einem einer Ammonoxidation nachgeschalteten Quench, einer Eindampfstufe oder einer Destillationskolonne kommen, wobei das Phthalodinitril z.B. jeweils als Schmelze über Sumpf dieser thermischen Trennapparate abgetrennt wird, wie z.B. in der deutschen Patentanmeldung Nr. 10341633.1 vom 10.09.03 (BASF AG) beschrieben.

Alternativ kann im erfindungsgemäßen Verfahren auch aufgeschmolzenes, zuvor als Feststoff vorliegendes PDN eingesetzt werden. Das Aufschmelzen kann z.B. mittels eines Extruders erfolgen.

Vorteil der Eindosierung des PDNs als Schmelze in den flüssig en Ammoniak ist, dass das Phthalodinitril bereits unmittelbar nach der Vermischung verdünnt und bei deutlich reduzierter Temperatur vorliegt, bevor es mit Xylylendiamin (aus dem optionalen Umlaufkreis oder im Hydrierreaktor gebildet) in Kontakt kommt, wodurch eine ungewünschte Reaktion zwischen Nitril und Produkt weitgehend unterdrückt werden kann. Außerdem kann die Schmelzedosierung bei einem Druck erfolgen, der kleiner ist als der Reaktordruck, wodurch eine kostengünstigere Schmelzepumpe eingesetzt werden kann. Die Lösung kann dann anschließend auf den gewünschten Reaktordruck komprimiert werden.

Das Zufahren und Lösen der Phthalodinitrilschmelze in flüssigem Ammoniak erfordert eine Mischeinrichtung, bevorzugt eine Mischdüse oder einen Mischbehälter.

Eine Mischdüse kann im einfachsten Fall durch ein Rohrleitungs-T-Stück realisiert werden. Bevorzugt weist der Düsenmund eine Verjüngung auf.

Bei Einsatz einer Mischdüse werden die Ströme getrennt zugeführt und im anschließenden Rohr auf Grund der herrschenden Turbulenz vermischt und homogenisiert. Vorteilhaft kann zusätzlich ein statischer Mischer nachgeschaltet werden. Es wird jedoch kein zusätzlicher Apparat, wie etwa ein Rührkessel zum Lösen von (festem oder flüssigem) Phthalodinitril in einem Lösungsmittel benötigt.

Bevorzugt ist die Mischeinrichtung am Ort der Phthalodinitrilzufuhr in den Strom von flüssigem Ammoniak auf eine Temperatur im Bereich von 1 bis 40°C, besonders im Bereich von 5 bis 25 °C, oberhalb des Schmelzpunktes des eingesetzten Phthalodinitrils beheizt.

Bevorzugt erfolgt die PDN-Zufuhr praktisch bei einem Absolutdruck zwischen 15 bar und dem Reaktordruck. Der Mindestdruck ergibt sich aus der besonders bevorzugten Randbedingung, dass bei der Vermischung und der sich einstellenden Mischtemperatur keine Verdampfung eintritt, sondern die Mischung flüssig bleibt. Er ist somit von der Ausgangstemperatur und dem Mengenverhältnis der zu mischenden Komponenten abhängig. Eine Vermischung bei niedrigem Druck, z.B. 40 bar, bietet den Vorteil, dass die Schmelzepumpe nicht für den deutlich höheren Reaktordruck ausgelegt werden muss. Die PDN-Lösung in Ammoniak muss in diesem Fall jedoch noch mittels einer - konstruktiv einfacheren - Hochdruckpumpe auf den Reaktordruck komprimiert werden. Auf letztere kann verzichtet werden, falls die Vermischung bereits bei Reaktordruck erfolgt. Welche Variante zu bevorzugen ist, richtet sich nach Verfügbarkeit der Apparate und Maschinen sowie nach wirtschaftlichen Gesichtspunkten.

Besonders bevorzugt wird mittels einer Mischdüse als Mischeinrichtung das flüssige Phthalodinitril in den Strom von flüssigem Ammoniak eingedüst.

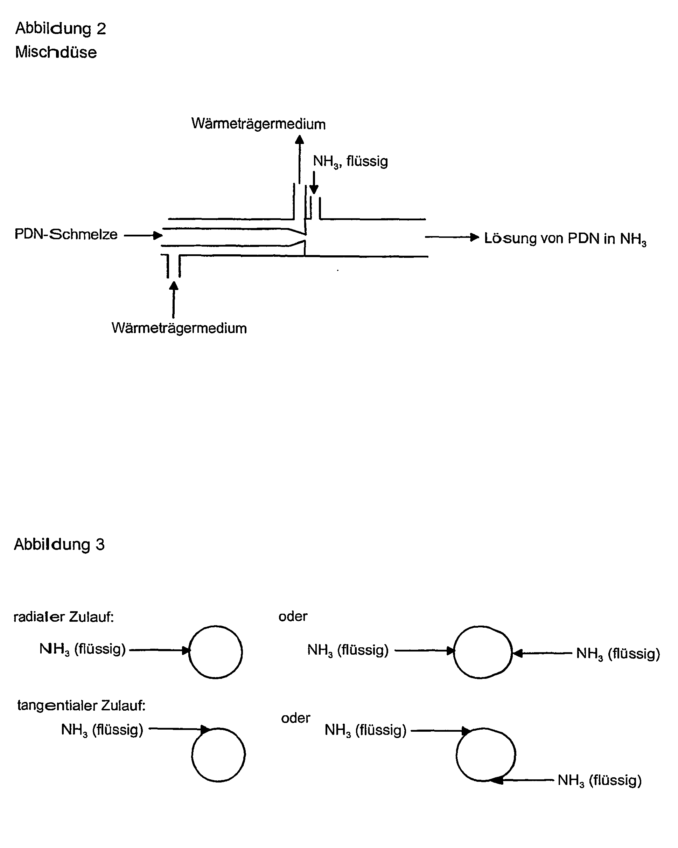

Eine bevorzugte Ausführungsform der Mischdüse ist in Abbildung 2 im Anhang dargestellt. Die Beheizung der Mischdüse kann z.B. mit Dampf, Wärmeträgeröl oder auch elektrisch erfolgen.

Der Zulauf des flüssigen Ammoniaks kann über einen oder mehrere radial oder tangential angebrachte Stutzen erfolgen, z.B. wie in der Abbildung 3 gezeigt.

Wichtig ist die lokal hohe Strömungsgeschwindigkeit (hoher Impulsstrom und Turbulenz), so dass eine schnelle Vermischung (Homogenisierung) eintritt. Bei laminarer Strömung reicht der Stoffaustausch zur Homogenisierung nicht aus und die Ströme werden nur unzureichend gemischt (Schlierenbildung).

Geeignete Mischdüsen sind z.B. in Ullmann's Encyclopedia of Industrial Chemistry, 5. Auflage, Vol. B4, Seiten 565 - 569, beschrieben.

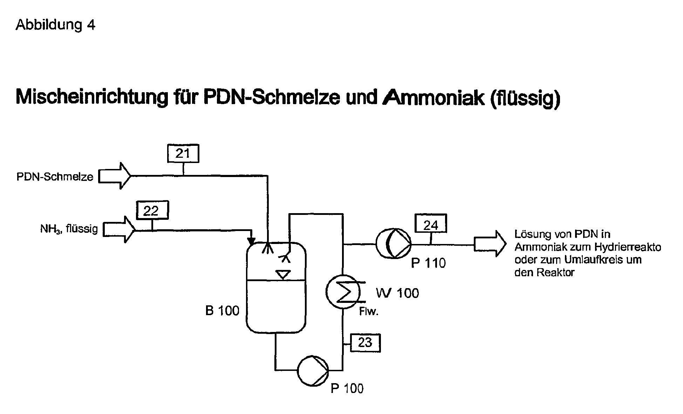

Eine weitere besonders bevorzugte Mischeinrichtung ist ein Mischbehälter mit Umlaufkreis, wie beispielsweise in Abbildung 4 gezeigt.
Die PDN-Schmelze (Strom [21]) und der flüssige Ammoniak (Strom [22]) werden getrennt dem Behälter B 100 zugeführt. PDN und die Lösung aus dem Umlaufkreis (Strom [23]) werden jeweils mit Hilfe von Düsen versprüht (verdüst). Dabei können für jeden Strom eine oder auch mehrere Düsen zum Einsatz kommen. Der flüssige Ammoniak kann ebenfalls versprüht werden. Falls auf den Umlaufkreis um den Mischbehälter verzichtet wird, wird Ammoniak an Stelle der Lösung versprüht. Die Lösung aus dem Umlaufkreis bzw. der flüssige Ammoniak werden dabei bevorzugt so versprüht, dass ein Großteil des Gasraumes erfasst wird. Insbesondere wird die Lösung bzw. der Ammoniak über die gesamte Querschnittsfläche versprüht, so dass ein Teil der Lösung bzw. ein Teil des flüssigen Ammoniaks an den Behälterwänden herunterläuft und einen Flüssigkeitsfilm bildet. Feststoffablagerungen werden so vermieden. Das PDN wird ebenfalls versprüht, jedoch bevorzugt innerhalb eines engeren Sprühkegels, der sich im wesentlichen innerhalb des Lösungs- bzw. Ammoniaksprühkegels befindet. Vorteilhaft kann das z.B. dadurch realisiert werden, dass die PDN Düse zentrisch angebracht ist und z.B. drei oder mehr Düsen für Lösung bzw. Ammoniak darum herum angeordnet sind. Die kleinen PDN Tröpfchen vermischen und lösen sich spontan im Ammoniak bzw. in der Lösung. Die Mischung wird mit der Pumpe P 100 aus dem Mischbehälter gefördert und kann in einem Wärmeübertrager W 100 auf die gewünschte Temperatur abgekühlt werden. Anschließend wird die Mischung mit der Pumpe P 110 auf Reaktordruck komprimiert (Strom [24]). Vorteilhaft wird ein Teil der Mischung nach dem Reaktor wieder zum Mischbehälter zurückgefahren und mittels Düsen versprüht. Es kann aber auch auf den Umlaufkreis verzichtet werden. Dann ist dem Mengenstrom [23] gleich dem Mengenstrom [24]. Der Betriebsdruck des Behälters ist von den Verfahrensbedingungen abhängig, insbesondere von der Temperatur. Bei gegebener Zulauftemperatur des frischen Ammoniaks (Strom [22]) und der PDN-Schmelze (Strom [21]) kann durch Variation des Umlaufstromes und Einstellung der Temperatur des Umlaufstromes nach dem Kühler W 100 die Mischtemperatur im Behälter innerhalb gewisser Grenzen, insbesondere unter Berücksichtigung der von der Temperatur abhängigen Löslichkeit von PDN in Ammoniak gewählt werden. Damit stellt sich dann über der Lösung im Gasraum der entsprechende Dampfdruck ein. Ggf. kann im Gasraum auch noch Inertgas, z.B. Stickstoff, anwesend sein. Vorteilhaft beträgt der Druck im Behälter B 100 nicht mehr als 25 bar, so dass kostengünstige Apparate und Maschinen zum Einsatz kommen können. Die Hochdruckpumpe P 110 komprimiert dann die Lösung auf den Reaktordruck für die Hydrierung. Je nachdem, ob die Hydrierung mit oder ohne Umlaufkreis um den Hydrierreaktor gefahren wird, kann der Strom [24] zum Reaktor oder in den Umlaufkreis um den Reaktor gefahren werden.

In einer bevorzugten Ausführungsform des erfindungsgemäßen Verfahrens wird ein Teil des Reaktoraustrags als flüssiger Umlaufstrom kontinuierlich zum Reaktoreingang zurückgeführt (Kreislauffahrweise) und die flüssige Mischung aus Ammoniak und Phthalodinitril in den Umlaufstrom um den Hydrierreaktor zugefahren, wobei der Umlaufstrom zu größer 93 Gew.-% aus flüssigem Ammoniak und Xylylendiamin besteht.

Durch Anwendung eines Kreislaufstromes kann die Phthalodinitrillösung weiter verdünnt oder die Menge an frischem Ammoniak reduziert werden. In jedem Fall wird im Reaktor eine hohe Ammoniakkonzentration erreicht, was sich wiederum günstig auf die Selektivität auswirkt.

Das erfindungsgemäße Verfahren ist in einer vorteilhaften Ausführungsform weiterhin dadurch gekennzeichnet, dass das Reaktorgemisch kein weiteres Lösungsmittel für Phthalodinitril enthält.

Der Phthalodinitril-Umsatz im Hydrierreaktor beträgt bei einfachem Durchgang bevorzugt größer 99 %, besonders größer 99,5 %, ganz besonders größer 99,9 %, insbesondere größer 99,95 %, ganz besonders größer 99,97 %. Im Hydrierreaktor wird also durch entsprechende Einstellung der Reaktionsbedingungen (Druck, Temperatur, Molverhältnisse von PDN, NH₃ und H₂, Katalysator, Mengenströme, Verweilzeit im Reaktor) praktisch Vollumsatz gefahren.

Bevorzugt besteht der flüssige Umlaufstrom um den Hydrierreaktor (gemäß der Verfahrensvariante mit Kreislauffahrweise) zu größer 94 Gew.-%, insbesondere größer 95 Gew.-%, ganz besonders größer 96 Gew.-%, aus flüssigem Ammoniak und Xylylendiamin; den Rest bilden Nebenkomponenten.

Nebenkomponenten im flüssigen Umlaufstrom um den Hydrierreaktor (Kreislaufstrom) können bei der Reaktion gebildete Nebenprodukte sowie gelöste Gase und mit dem Phthalodinitril zugefahrene Nebenkomponenten, jedoch kein weiteres Lösungsmittel, z.B. organisches Lösungsmittel, für Phthalodinitril sein.

Der Umlaufstrom um den Hydrierreaktor (gemäß der Verfahrensvariante mit Kreislauffahrweise) enthält bevorzugt im Bereich von 25 bis 90 Gew.-%, besonders 30 bis 70 Gew.-%, insbesondere 45 bis 60 Gew.-%, flüssigen Ammoniak.

Der Teil des flüssigen Reaktoraustrags, der (gemäß der Verfahrensvariante mit Kreislauffahrweise) als Umlaufstrom kontinuierlich zum Reaktoreing ang zurückgeführt wird, macht bevorzugt 20 bis 95 Gew.-%, besonders 50 bis 92 Gew.-%, insbesondere 75 bis 90 Gew.-%, des gesamten flüssigen Reaktoraustrags aus.

Das Gewichtsverhältnis von Phthalodinitril/Ammoniak-Zulaufstrom zu Umlaufstrom um den Hydrierreaktor (gemäß der Verfahrensvariante mit Kreislauffahrweise) liegt bevorzugt im Bereich von 0,05 bis 5, besonders im Bereich von 0,1 bis 2,0, insbesondere im Bereich von 0,15 bis 1,0.

Die Reaktionstemperatur liegt bevorzugt im Bereich von 40 bis 150°C, besonders bevorzugt 60 bis 135°C, insbesondere 70 bis 130°C.

Die Ammoniakmenge, die Menge des optional vorhandenen Kreislaufstromes um den Hydrierreaktor (gemäß der Verfahrensvariante mit Kreislauffahrweise) und die Reaktorzulauftemperatur werden so eingestellt, dass die Reaktoraustrittstemperatur den gewünschten Maximalwert (z.B. 130°C) nicht überschreitet, da mit zunehmender Temperatur verstärkt Nebenprodukte gebildet werden. Die Reaktor-zulauftemperatur wird so eingestellt (z.B. durch einen zusätzlichen Wärmeübertrager oder, bevorzugt, durch geeignete Einstellung der Temperatur der zu mischenden Ströme), dass die Reaktion hinreichend schnell verläuft und Vollumsatz erreicht wird. Durch Variation von Kreislaufmengenstrom um den Hydrierreaktor bzw. Frischmengenstrom an Ammoniak ist es somit möglich, sowohl Eintritts- als auch Austrittstemperatur des Reaktors einzustellen und optimal an die ablaufenden Reaktionen anzupassen und so die XDA-Ausbeute zu optimieren.

Die Hydrierung wird bevorzugt bei einem Absolutdruck im Bereich von 100 bis 300 bar, insbesondere 120 bis 220 bar, ganz besonders 150 bis 200 bar, durchgeführt.

Für die Hydrierung können dem Fachmann bekannte Katalysatoren und Reaktoren (insbesondere Rohrreaktoren oder Rohrbündelreaktoren; Festbett- oder Suspensionsfahrweise) angewendet werden.

Bei der Katalysatorfestbettfahrweise ist sowohl die Sumpf- als auch die Rieselfahrweise möglich. Bevorzugt ist eine Rieselfahrweise.

Bevorzugt wird der Reaktor adiabat betrieben, während die entstehende Reaktionswärme über einen im Umlaufkreis eingebauten Kühler sowie optional mit dem verwendeten Kreisgas abgeführt wird. Dies erhöht zusätzlich die Selektivität der Reaktion durch die weitere Unterdrückung von Nebenprodukten.
Alternativ ist aber auch ein gekühlter Reaktor, beispielsweise ein Rohrbündelreaktor, einsetzbar.

Bevorzugt sind Katalysatoren, die Kobalt und/oder Nickel und/oder Eisen, als Volikatalysator oder auf einem inerten Träger, enthalten.
Besonders bevorzugt wird die Hydrierung an einem Mangan-dotierten Cobalt-Vollkatalysator durchgeführt.

Geeignete Katalysatoren sind beispielsweise Raney-Nickel, Raney-Cobalt, Co-Vollkontakt, Titan-dotiertes Cobalt auf Träger (JP-A-2002 205980), Ni auf SiO₂-Träger (WO-A-2000/046179), Co/Ti/Pd auf SiO₂-Träger (CN-A-1 285 343, CN-A-1 285 236) und Nickel und/oder Cobalt auf Zirkoniumdioxid-Träger (EP-A1-1 262 232).

Beispiele für weitere geeignete Katalysatoren finden sich z.B. in den Anmeldungen GB-A-852,972 (Äquivalent: DE-A-11 19 285) (BASF AG), DE-A-12 59 899 (BASF AG) und den US Patenten Nr. 3,069,469 (California Research Corp.) und 4,482,741 (UOP Inc.).

Besonders bevorzugte Katalysatoren sind die in EP-A1-742 045 (BASF AG) offenbarten Cobalt-Vollkontakte, dotiert mit Mn, P, und Alkalimetall (Li, Na, K, Rb, Cs).
Die katalytisch aktive Masse dieser Katalysatoren besteht vor der Reduktion mit Wasserstoff aus 55 bis 98 Gew.-%, insbesondere 75 bis 95 Gew.-%, Cobalt, 0,2 bis 15 Gew.% Phosphor, 0,2 bis 15 Gew.-% Mangan und 0,05 bis 5 Gew.-% Alkalimetall, insbesondere Natrium, jeweils berechnet als Oxid.

Weitere geeignete Katalysatoren sind die in EP-A-963 975 (BASF AG) offenbarten Katalysatoren, deren katalytisch aktive Masse vor der Behandlung mit Wasserstoff 22 bis 40 Gew.-% ZrO₂,
1 bis 30 Gew.-% sauerstoffhaltige Verbindungen des Kupfers, berechnet als CuO,
15 bis 50 Gew.-% sauerstoffhaltige Verbindungen des Nickels, berechnet als NiO, wobei das molare Ni : Cu-Verhältnis größer 1 ist,
15 bis 50 Gew.-% sauerstoffhaltige Verbindungen des Kobalts, berechnet als CoO,
0 bis 10 Gew.-% sauerstoffhaltige Verbindungen des Aluminiums und/oder Mangans, berechnet als Al₂O₃ bzw. MnO₂,
und keine sauerstoffhaltigen Verbindungen des Molybdäns enthält,
beispielsweise der in loc. cit., Seite 17, offenbarte Katalysator A mit der Zusammensetzung 33 Gew.-% Zr, berechnet als ZrO₂, 28 Gew.-% Ni, berechnet als NiO, 11 Gew.-% Cu, berechnet als CuO und 28 Gew.-% Co, berechnet als CoO,
die
in EP-A-696 572 (BASF AG) offenbarten Katalysatoren, deren katalytisch aktive Masse vor der Reduktion mit Wasserstoff 20 bis 85 Gew.-% ZrO₂, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als MoO₃, und O bis 10 Gew.-% sauerstoffhaltige Verbindungen des Aluminiums und/oder Mangans, berechnet als Al₂O₃ bzw. MnO₂, enthält, beispielsweise der in loc. cit., Seite 8, offenbarte Katalysator mit der Zusammensetzung 31,5 Gew.-% ZrO₂, 50 Gew.-% NiO, 17 Gew.% CuO und 1,5 Gew.-% MoO₃,
und die in WO-A-99/44984 (BASF AG) beschriebenen Katalysatoren enthaltend (a) Eisen oder eine Verbindung auf der Basis von Eisen oder deren Ge mische, (b) von 0,001 bis 0,3 Gew.-% bezogen auf (a) eines Promoters auf der Basis von 2, 3, 4 oder 5 Elementen ausgewählt aus der Gruppe Al, Si, Zr, Ti, V, (c) von 0 bis 0,3 Gew.-% bezogen auf (a) einer Verbindung auf der Basis eines Alkali- und/oder Erdalkalimetalls, sowie (d) von 0,001 bis 1 Gew.-% bezogen auf (a) Mangan.

Das erfindungsgemäße Verfahren lässt sich z.B. wie folgt ausführen:

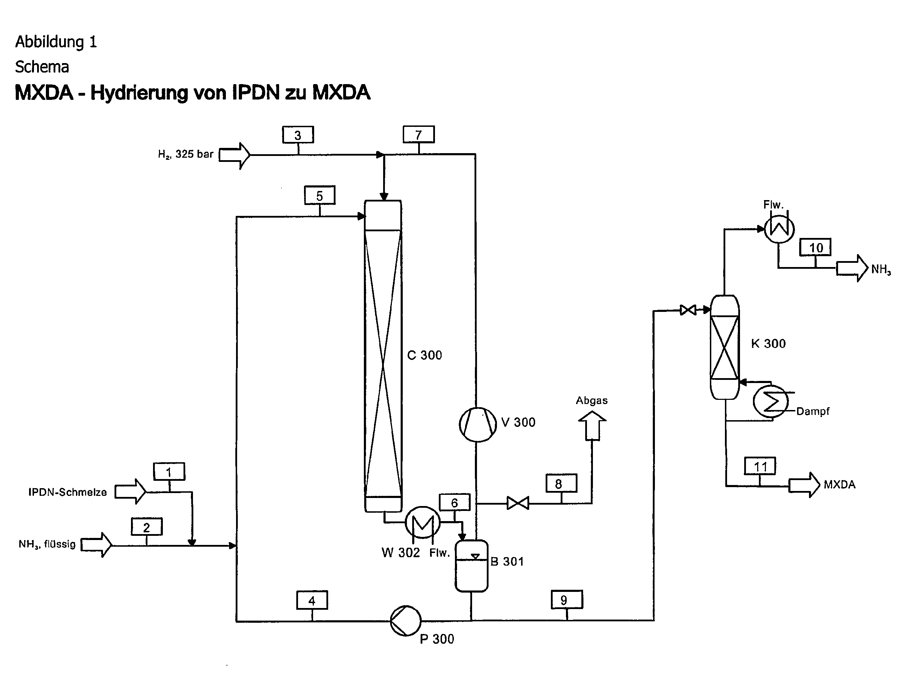

In Abbildung 1 ist eine mögliche Anordnung des Hydrierreaktors einschließlich des optionalen Umlaufkreises und eines Wärmeübertragers dargestellt. Die Phthalodinitrilschmelze wird als Strom [1] zugeführt und mit dem flüssigen Ammoniak [2] gemischt. Die Mischung wird entweder mit dem optional vorhandenen Umlaufkreis [4] gemischt oder direkt dem Reaktor zugefahren. Zum besseren Vermischen mit dem Umlaufkreis kann z.B. ein statischer Mischer verwendet werden.
Wasserstoff und ggf. Kreisgas können mittels eines optionalen Wärmeübertragers auf die gewünschte Reaktorzulauftemperatur erwärmt werden. Gas und Flüssigkeit können gemeinsam oder - bevorzugt - getrennt dem Reaktor zugefahren werden. Bevorzugt wird die Temperatur der zu mischenden Ströme mittels Wärmeübertragern so eingestellt, dass hinter dem Mischpunkt kein Wärmeübertrager mehr erforderlich ist. Im Reaktor erfolgt die Hydrierung praktisch quantitativ, so dass im Reaktionsaustrag praktisch kein Phthalodinitril mehr vorhanden ist. Der Reaktionsaustrag kann dann abgekühlt werden, und Gas und Flüssigkeit werden unter Druck in einem Hochdruckabscheider getrennt. Bei Kreisflüssigkeitsfahrweise wird ein Teil der Flüssigkeit aus dem Reaktionsaustrag ohne Aufarbeitung im Kreis gefahren (Strom [4]). Ansonsten wird der Reaktionsaustrag der Aufarbeitung (Strom [9]) zugeführt. Ein Teil des Gases wird ausgeschleust, um die Aufpegelung von Inerten (CO, N₂, CH₄, Edelgase, etc.) zu vermeiden. Der größte Teil des Gases wird über einen Verdichter zum Reaktoreingang zurückgeführt. Bei nicht zu hohem Druckverlust im Reaktor kann hierzu bevorzugt auch eine Ejektorstrahldüse ("Wasserstrahlpumpe") verwendet werden. Insgesamt kann die Kreisgasmenge in weiten Bereichen variiert werden, etwa vom mehrfachen der Frischgasmenge bis hin zu Null (Fahrweise ohne Kreisgas). Die Kreisgasfahrweise ist günstig, um den Reaktor für einen guten Stoffübergang ausreichend gasseitig zu belasten und um für Inertgase einen hinreichenden Schleppstrom zur Verfügung zu stellen, um sie am Reaktorausgang ausschleusen zu können. Zusätzlich kann ein Teil der Reaktionswärme mit dem Gasstrom abgeführt werden. Mit zunehmender Temperatur verdampft eine zunehmende Menge an Ammoniak, was den kühlenden Effekt des Kreisgases noch verstärkt. Der Reaktionsaustrag (Strom [9]) wird dann zunächst einer Druckdestillation zugeführt, in der flüssiger Ammoniak über Kopf (Strom [10]) und weitgehend ammoniakfreies, rohes Xylylendiamin über Sumpf (Strom [11]) gewonnen werden, wobei der Ammoniak in kondensierter Form wieder der Hydrierstufe zugeführt werden kann. Das rohe Xylylendiamin wird z.B. durch Destillation weiter gereinigt.

Im erfindungsgemäßen Verfahren kann das Gewichtsverhältnis der Frischzuläufe von Dinitril und Ammoniak (z.B. gem. Abbildung 1 das Verhältnis von Strom [1] zu Strom [2]) um so größer gewählt werden, je größer der Kreislaufstrom (gemäß der Verfahrensvariante mit Kreislauffahrweise) ist. Der Ammoniakmengenstrom ist nach unten hin durch die Löslichkeit von PDN in flüssigem Ammoniak bei der gegebenen Temperatur begrenzt (z.B. beträgt die Löslichkeit von IPDN in NH₃ bei 40°C ca. 45 Gew.-%.). Bevorzugt beträgt das Einsatzstoff-Gewichtsverhältnis von Dinitril zu Ammoniak 1 : 0,5 bis 1 : 10, vorzugsweise 1 : 0,6 bis 1 : 5, besonders bevorzugt 1:0,9 bis 1 : 3,5.

Bei der Fahrweise ohne Kreisflüssigkeit um den Hydrierreaktor beträgt das Einsatzstoff-Gewichtsverhältnis von Dinitril zu Ammoniak bevorzugt 1 : 2,5 bis 1 : 100, vorzugsweise 1 : 4 bis 1 : 13 , besonders bevorzugt 1 : 5 bis 1 : 10.

### Isolierung des XDAs:

Nach der Hydrierung wird der eingesetzte Ammoniak abgetrennt, z.B. abdestilliert.

Bevorzugt erfolgt eine Reinigung des Xylylendiamins durch Abdestillation leichtersiedender Nebenprodukte (bei gleichem Druck) über Kopf und destillativer Abtrennung von schwerersiedenden Verunreinigungen über Sumpf.

Besonders bevorzugt ist die Fahrweise, in der man nach der Hydrierung den Ammoniak sowie gegebenenfalls leichtsiedende Nebenprodukte über Kopf abdestilliert und danach schwerersiedende Verunreinigungen vom Xylylendiamin destillativ über Sumpf abtrennt.

In einer besonderen Ausführungsform kann die Abtrennung leichter- und schwerersiedender Nebenprodukte auch in einer Seitenabzugs- oder Trennwandkolonne erfolgen, wobei reines Xylylendiamin über einen flüssigen oder gasförmigen Seitenabzug gewonnen wird.

Je nach gewünschter Reinheit wird das Produkt (XDA) zusätzlich mit einem organischen Lösungsmittel, bevorzugt einem aliphatischen Kohlenwasserstoff, insbesondere einem cycloaliphatischen Kohlenwasserstoff, ganz besonders Cyclohexan oder Methylcyclohexan, extrahiert.
Diese Reinigung durch Extraktion kann z.B. gemäß DE-A-1 074 592 (BASF AG) erfolgen.

### Beispiele

### Beispiel 1:

90 g/h geschmolzenes IPDN (kommerzielles, geschupptes IPDN, welches durch Erhitzen auf ca. 170°C aufgeschmolzen wurde) wurden mittels eines Rohrleitungs-T-Stücks mit 90 g/h Frisch-Ammoniak gelöst. Dies entspricht der Löslichkeit bei 45°C. Die berechnete Mischtemperatur bei idealer Vermischung beträgt 74°C, wenn Ammoniak bei 25°C zudosiert wird. Der Siededruck beträgt 32,3 bar (abs.), d.h. oberhalb dieses Druckes bleibt die Mischung flüssig und es findet keine Verdampfung von Ammoniak statt. Die Lösung wurde in einen Kreislaufstrom (ca. 1000 g/h) bestehend aus dem flüssigen Rückführstrom des Reaktoraustrags gefahren. Die erhaltene Reaktionsmischung wurde kontinuierlich in einen Rohreaktor an einem Kobalt-Vollkontakt bei 90°C und 200 bar hydriert. Der abgezogene Teil des Reaktoraustrags wurde in einer Ammoniakkolonne vom Großteil der Ammoniakmenge befreit und über GC-u ntersucht. Bei Vollumsatz des eingesetzten IPDN (d.h. Umsatz größer 99,95 %; per GC kein Edukt mehr nachweisbar) lag die Selektivität bei 93 %.

In anschließenden Destillationsschritten wurden zuerst Rest-Ammoniak und leichtsiedende Nebenkomponenten abgetrennt. Nach Abtrennung der hochsiedenden Verunreinigungen über Sumpf wurde MXDA als Kopfprodukt einer Destillationskolonne in einer Reinheit von mehr als 99,9 Gew.-% erhalten.

### Beispiel 2:

90 g/h geschmolzenes IPDN (kommerzielles, geschupptes IPDN, welches durch Erhitzen auf ca. 170°C aufgeschmolzen wurde) wurden mittels eines Rohrleitungs-T-Stücks mit 270 g/h Frisch-Ammoniak gelöst. Die berechnete Mischtemperatur bei idealer Vermischung beträgt 52°C, wenn Ammoniak bei 25°C zudosiert wird. Der Siededruck beträgt 20,5 bar (abs.), d.h. oberhalb dieses Druckes bleibt die Mischung flüssig und es findet keine Verdampfung von Ammoniak statt. Die Lösung wurde in einen Kreislaufstrom (ca. 900 g/h) bestehend aus dem flüssigen Rückführstrom des Reaktoraustrags gefahren. Die erhaltene Reaktionsmischung wurde kontinuierlich in einen Rohreaktor an einem Kobalt-Vollkontakt bei 90°C und 200 bar hydriert. Der abgezogene Teil des Reaktoraustrags wurde in einer Ammoniakkolonne vom Großteil der Ammoniakmenge befreit und über GC-untersucht. Bei Vollumsatz des eingesetzten IPDN lag die Selektivität bei 95 %.

In anschließenden Destillationsschritten wurden zuerst Rest-Ammoniak und leichtsiedende Nebenkomponenten abgetrennt. Nach Abtrennung der hochsiedenden Verunreinigungen über Sumpf wurde MXDA als Kopfprodukt einer Destillationskolonne in einer Reinheit von mehr als 99,9 Gew.-% erhalten.

### Beispiel 3:

90 g/h geschmolzenes IPDN (kommerzielles, geschupptes IPDN, welches durch Erhitzen auf ca. 170°C aufgeschmolzen wurde) wurden mittels eines Rohrleitungs-T-Stücks mit 600 g/h Frisch-Ammoniak gelöst. Die berechnete Mischtemperatur bei idealer Vermischung beträgt 41 °C, wenn Ammoniak bei 25°C zudosiert wird. Der Siededruck beträgt 16 bar (abs.), d.h. oberhalb dieses Druckes bleibt die Mischung flüssig und es findet keine Verdampfung von Ammoniak statt. Die erhaltene Reaktionsmischung wurde kontinuierlich in einen Rohreaktor an einem Kobalt-Vollkontakt bei 90°C und 200 bar hydriert. Der abgezogene Teil des Reaktoraustrags wurde in einer Ammoniakkolonne vom Großteil der Ammoniakmenge befreit und über GC-untersucht. Bei Vollumsatz des eingesetzten IPDN lag die Selektivität bei 93 %.

In anschließenden Destillationsschritten wurden zuerst Rest-Ammoniak und leichtsiedende Nebenkomponenten abgetrennt. Nach Abtrennung der hochsiedenden Verunreinigungen über Sumpf wurde MXDA als Kopfprodukt einer Destillationskolonne in einer Reinheit von mehr als 99,9 Gew.-% erhalten.

## Patentansprüche

1. Verfahren zur Herstellung von Xylylendiamin durch kontinuierliche Hydrierung von flüssigem Phthalodinitril an einem Heterogenkatalysator in Gegenwart von flüssigem Ammoniak in einem Reaktor, **dadurch gekennzeichnet, dass** mittels einer Mischeinrichtung ein Strom einer Phthalodinitrilschmelze flüssig mit einem Strom von flüssigem Ammoniak gemischt wird und die flüssige Mischung in den Hydrierreaktor gefahren wird.

2. Verfahren nach Anspruch 1 zur Herstellung von meta-Xylylendiamin durch Hydrierung von Isophthalodinitril.

3. Verfahren nach den Ansprüchen 1 oder 2, **dadurch gekennzeichnet, dass** die Mischeinrichtung am Ort der Phthalodinitrilzufuhr in den Strom von flüssigem Ammoniak auf eine Temperatur im Bereich von 1 bis 40°C oberhalb des Schmelzpunktes des eingesetzten Phthalodinitrils beheizt ist.

4. Verfahren nach einem der vorhergehenden Ansprüche, **dadurch gekennzeichnet, dass** mittels einer Mischdüse als Mischeinrichtung das flüssige Phthalodinitril in den Strom von flüssigem Ammoniak eingedüst wird.

5. Verfahren nach einem der Ansprüche 1 bis 3, **dadurch gekennzeichnet, dass** in einem Mischbehälter als Mischeinrichtung das flüssige Phthalodinitril und flüssiger Ammoniak oder eine Lösung von Phthalodinitril in Ammoniak verdüst werden.

6. Verfahren nach einem der vorhergehenden Ansprüche, **dadurch gekennzeichnet, dass** ein Teil des Reaktoraustrags als flüssiger Umlaufstrom kontinuierlich zum Reaktoreingang zurückgeführt wird (Kreislauffahrweise) und die flüssige Mischung aus Ammoniak und Phthalodinitril in den Umlaufstrom um den Hydrierreaktor zugefahren wird, wobei der Umlaufstrom zu größer 93 Gew.-% aus flüssigem Ammoniak und Xylylendiamin besteht.

7. Verfahren nach einem der vorhergehenden Ansprüche, **dadurch gekennzeichnet, dass** das Reaktorgemisch kein weiteres Lösungsmittel für Phthalodinitril enthält.

8. Verfahren nach einem der vorhergehenden Ansprüche, **dadurch gekennzeichnet, dass** der Phthalodinitril-Umsatz im Hydrierreaktor bei einfachem Durchgang größer 99 % beträgt.

9. Verfahren nach einem der Ansprüche 1 bis 7, **dadurch gekennzeichnet, dass** der Phthalodinitril-Umsatz im Hydrierreaktor bei einfachem Durchgang größer 99,5 % beträgt.

10. Verfahren nach einem der Ansprüche 6 bis 9, **dadurch gekennzeichnet, dass** der Umlaufstrom um den Hydrierreaktor zu größer 94 Gew.-% aus flüssigem Ammoniak und Xylylendiamin besteht.

11. Verfahren nach einem der Ansprüche 6 bis 10, **dadurch gekennzeichnet, dass** der Umlaufstrom um den Hydrierreaktor im Bereich von 25 bis 90 Gew.-% flüssigen Ammoniak enthält.

12. Verfahren nach einem der Ansprüche 6 bis 11, **dadurch gekennzeichnet, dass** der Teil des flüssigen Reaktoraustrags, der als Umlaufstrom kontinuierlich zum Reaktoreingang zurückgeführt wird, 20 bis 95 Gew.-% des gesamten flüssigen Reaktoraustrags ausmacht.

13. Verfahren nach einem der Ansprüche 6 bis 12, **dadurch gekennzeichnet, dass** das Gewichtsverhältnis von Phthalodinitril/Ammoniak-Zulaufstrom zu Umlaufstrom um den Hydrierreaktor im Bereich von 0,05 bis 5 liegt.

14. Verfahren nach einem der vorhergehenden Ansprüche, **dadurch gekennzeichnet, dass** die Hydrierung bei einer Temperatur im Bereich von 40 bis 150°C durchgeführt wird.

15. Verfahren nach einem der vorhergehenden Ansprüche, **dadurch gekennzeichnet, dass** die Hydrierung bei einem Absolutdruck im Bereich von 100 bis 300 bar durchgeführt wird.

16. Verfahren nach einem der vorhergehenden Ansprüche, **dadurch gekennzeichnet, dass** die Hydrierung an einem Katalysator enthaltend Ni, Co und/oder Fe, als Vollkatalysator oder auf einem inerten Träger, durchgeführt wird.

17. Verfahren nach einem der vorhergehenden Ansprüche, **dadurch gekennzeichnet, dass** die Hydrierung an einem Mangan-dotierten Cobalt-Vollkatalysator durchgeführt wird.

18. Verfahren nach einem der vorhergehenden Ansprüche, **dadurch gekennzeichnet, dass** der Katalysator in einem Rohrreaktor oder Rohrbündelreaktor als Festbett angeordnet ist.

19. Verfahren nach dem vorhergehenden Anspruch, **dadurch gekennzeichnet, dass** der Reaktor in Rieselfahrweise betrieben wird.

20. Verfahren nach einem der vorhergehenden Ansprüche, **dadurch gekennzeichnet, dass** der Reaktor adiabat betrieben wird.

21. Verfahren nach einem der Ansprüche 6 bis 20, **dadurch gekennzeichnet, dass** dem Umlaufstrom um den Hydrierreaktor in einem Kühler Wärme entzogen wird.

22. Verfahren nach einem der vorhergehenden Ansprüche, **dadurch gekennzeichnet, dass** nach der Hydrierung eine Reinigung des Xylylendiamins durch Abdestillation des Ammoniaks sowie gegebenenfalls leichtersiedender Nebenprodukte über Kopf und destillativer Abtrennung von schwerersiedenden Verunreinigungen über Sumpf erfolgt.

23. Verfahren nach dem vorhergehenden Anspruch, **dadurch gekennzeichnet, dass** das Xylylendiamin nach der Destillation zur weiteren Reinigung mit einem organischem Lösungsmittel extrahiert wird.

24. Verfahren nach dem vorhergehenden Anspruch, **dadurch gekennzeichnet, dass** man zur Extraktion Cyclohexan oder Methylcyclohexan verwendet.

## Claims

1. A process for preparing xylylenediamine by continuously hydrogenating liquid phthalonitrile over a heterogeneous catalyst in the presence of liquid ammonia in a reactor, which comprises mixing a stream of a phthalonitrile melt in liquid form by means of a mixer unit with a stream of liquid ammonia and conducting the liquid mixture into the hydrogenation reactor.

2. The process according to claim 1 for preparing meta-xylylenediamine by hydrogenating isophthalonitrile.

3. The process according to claims 1 or 2, wherein the mixer unit is heated at the point of the phthalonitrile supply into the stream of liquid ammonia to a temperature in the range from 1 to 40°C above the melting point of the phthalonitrile used.

4. The process according to any of the preceding claims, wherein the liquid phthalonitrile is sprayed into the stream of liquid ammonia by means of a mixer nozzle as the mixer unit.

5. The process according to any of claims 1 to 3, wherein the liquid phthalonitrile and liquid ammonia or a solution of phthalonitrile in ammonia are atomized in a mixer vessel as the mixer unit.

6. The process according to any of the preceding claims, wherein a portion of the reactor effluent is recycled as a liquid circulation stream continuously to the reactor inlet (circulation mode) and the liquid mixture of ammonia and phthalonitrile is fed into the circulation stream around the hydrogenation reactor, the circulation stream consisting to an extent of greater than 93% by weight of liquid ammonia and xylylenediamine.

7. The process according to any of the preceding claims, wherein the reactor mixture does not comprise any further solvent for phthalonitrile.

8. The process according to any of the preceding claims, wherein the phthalonitrile conversion in the hydrogenation reactor on single pass is greater than 99%.

9. The process according to any of claims 1 to 7, wherein the phthalonitrile conversion in the hydrogenation reactor on single pass is greater than 99.5%.

10. The process according to any of claims 6 to 9, wherein the circulation stream consists to an extent of greater than 94% by weight of liquid ammonia and xylylenediamine.

11. The process according to any of claims 6 to 10, wherein the circulation stream around the hydrogenation reactor comprises in the range from 25 to 90% by weight of liquid ammonia.

12. The process according to any of claims 6 to 11, wherein the portion of the liquid reactor effluent which is recycled as the circulation stream continuously to the reactor inlet makes up from 20 to 95% by weight of the overall liquid reactor effluent.

13. The process according to any of claims 6 to 12, wherein the weight ratio of phthalonitrile/ammonia feed stream to circulation stream around the hydrogenation reactor is in the range from 0.05 to 5.

14. The process according to any of the preceding claims, wherein the hydrogenation is carried out at a temperature in the range from 40 to 150°C.

15. The process according to any of the preceding claims, wherein the hydrogenation is carried out at an absolute pressure in the range from 100 to 300 bar.

16. The process according to any of the preceding claims, wherein the hydrogenation is carried out over a catalyst comprising Ni, Co and/or Fe, as an unsupported catalyst or on an inert support.

17. The process according to any of the preceding claims, wherein the hydrogenation is carried out over a manganese-doped unsupported cobalt catalyst.

18. The process according to any of the preceding claims, wherein the catalyst is disposed as a fixed bed in a tubular reactor or tube bundle reactor.

19. The process according to the preceding claim, wherein the reactor is operated in trickle mode.

20. The process according to any of the preceding claims, wherein the reactor is operated adiabatically.

21. The process according to any of claims 6 to 20, wherein heat is withdrawn from the circulation stream around the hydrogenation reactor in a cooler.

22. The process according to any of the preceding claims, wherein the xylylenediamine is purified after the hydrogenation by distilling off the ammonia and also any relatively low-boiling by-products overhead and distillatively removing relatively high-boiling impurities via the bottom.

23. The process according to the preceding claim, wherein the xylylenediamine is extracted after the distillation with an organic solvent for further purification.

24. The process according to the preceding claim, wherein cyclohexane or methylcyclohexane are used for the extraction.

## Revendications

1. Procédé de préparation de xylylènediamine par hydrogénation en continu de phtalodinitrile liquide sur un catalyseur hétérogène en présence d'ammoniaque liquide dans un réacteur, **caractérisé en ce qu'**un courant d'une fonte de phtalodinitrile est mélangée à l'état liquide au moyen d'un dispositif de mélange avec un courant d'ammoniaque liquide et le mélange liquide est amené dans le réacteur d'hydrogénation.

2. Procédé selon la revendication 1 pour la préparation de métaxylylènediamine par hydrogénation d'isophtalodinitrile

3. Procédé selon les revendications 1 ou 2, **caractérisé en ce que** le dispositif de mélange est chauffé au point d'apport de phtalodinitrile dans le courant d'ammoniaque liquide à une température dans la plage de 1 à 40°C au-dessus du point de fusion du phtalodinicrile utilisé.

4. Procédé selon l'une quelconque des revendications précédentes, **caractérisé en ce que** le phtalodinitrile liquide est injecté dans le courant d'ammoniaque liquide au moyen d'une buse de mélange en tant que dispositif de mélange.

5. Procédé selon l'une quelconque des revendications 1 à 3, **caractérisé en ce que** le phtalodinitrile liquide et l'ammoniaque liquide ou une solution de phtalodinitrile dans de l'ammoniaque est/sont pulvérisée/s dans un bac de mélange en tant que dispositif de mélange.

6. Procédé selon l'une quelconque des revendications précédentes, **caractérisé en ce qu'**une partie de l'effluent de réacteur est remise en circulation à l'entrée du réacteur de manière continue en tant que courant circulant liquide (procédé cyclique) et le mélange liquide d'ammoniaque et de phtalodinitrile est amené dans le courant circulant autour du réacteur d'hydrogénation, le courant circulant étant constitué pour plus de 93 % en poids d'ammoniaque liquide et de xylylènediamine.

7. Procédé selon l'une quelconque des revendications précédentes, **caractérisé en ce que** le mélange du réacteur ne contient aucun autre solvant pour le phtalodinitrile.

8. Procédé selon l'une quelconque des revendications précédentes, **caractérisé en ce que** la conversion du phtalodinitrile dans le réacteur d'hydrogénation pour un passage simple est supérieure à 99 %.

9. Procédé selon l'une quelconque des revendications 1 à 7, **caractérisé en ce que** la conversion du phtalodinitrile dans le réacteur d'hydrogénation dans le cas d'un passage simple est supérieure à 99,5 %.

10. Procédé selon l'une quelconque des revendications 6 à 9, **caractérisé en ce que** le courant circulant autour du réacteur d'hydrogénation est constitué pour plus de 94 % en poids d'ammoniaque liquide et de xylylènediamine.

11. Procédé selon l'une quelconque des revendications 6 à 10, **caractérisé en ce que** le courant circulant autour du réacteur d'hydrogénation contient de l'ammoniaque liquide dans la plage de 25 à 90 % en poids.

12. Procédé selon l'une quelconque des revendications 6 à 11, **caractérisé en ce que** la part de l'effluent liquide de réacteur, qui est remise en circulation de manière continue à l'entrée du réacteur en tant que courant circulant, constitue 20 à 95 % en poids de la totalité de l'effluent liquide de réacteur.

13. Procédé selon l'une quelconque des revendications 6 à 12, **caractérisé en ce que** le rapport de poids du courant d'alimentation de phtalodinitrile/ammoniaque sur le courant circulant autour du réacteur d'hydrogénation se situe dans la plage de 0,05 à 5.

14. Procédé selon l'une quelconque des revendications précédentes, **caractérisé en ce que** l'hydrogénation est mise en oeuvre à une température dans la plage de 40 à 150°C.

15. Procédé selon l'une quelconque des revendications précédentes, **caractérisé en ce que** l'hydrogénation est mise en oeuvre à une pression absolue dans la plage de 100 à 300 bar.

16. Procédé selon l'une quelconque des revendications précédentes, **caractérisé en ce que** l'hydrogénation est mise en oeuvre sur un catalyseur contenant Ni, Co et/ou Fe, en tant que catalyseur plein ou sur un support inerte.

17. Procédé selon l'une quelconque des revendications précédentes, **caractérisé en ce que** l'hydrogénation est mise en oeuvre sur un catalyseur plein au cobalt dopé au manganèse.

18. Procédé selon l'une quelconque des revendications précédentes, **caractérisé en ce que** le catalyseur est placé en tant que lit fixe dans un réacteur tubulaire ou un réacteur multitubulaire.

19. Procédé selon l'une quelconque des revendications précédentes, **caractérisé en ce que** le réacteur fonctionne selon un procédé de ruissellement.

20. Procédé selon l'une quelconque des revendications précédentes, **caractérisé en ce que** le réacteur fonctionne de manière adiabatique.

21. Procédé selon l'une quelconque des revendications 6 à 20, **caractérisé en ce que** de la chaleur est retirée du courant circulant autour du réacteur d'hydrogénation grâce à un radiateur.

22. Procédé selon l'une quelconque des revendications précédentes, **caractérisé en ce qu'**une purification de la xylylènediamine a lieu après l'hydrogénation grâce à une séparation par distillation de l'ammoniaque ainsi qu'éventuellement de produits secondaires à bas point d'ébullition par la tête et isolement par distillation d'impuretés à haut point d'ébullition par le bas.

23. Procédé selon la revendication précédente, **caractérisé en ce que** la xylylènediamine est extraite avec un solvant organique après la distillation pour purification ultérieure.

24. Procédé selon la revendication précédente, **caractérisé en ce que** du cyclohexane ou du méthylcyclohexane est utilisé pour l'extraction.
